# EUROPÄISCHE PATENTSCHRIFT

(11) **EP 1 709 916 B1**
(45) Veröffentlichungstag und Bekanntmachung des Hinweises auf die Patenterteilung: **12.12.2007**
(21) Anmeldenummer: 05007787.4
(22) Anmeldetag: 08.04.2005
(51) Int. Cl.: A61B 17/34, A61B 5/00

(54) **Vorrichtung zur Platzierung einer Sonde in lebendem Gewebe**
Device for placing a probe in living tissue
Dispositif pour la mise en place d'une sonde dans des tissus vivants

(43) Veröffentlichungstag der Anmeldung: 11.10.2006
(73) Patentinhaber: F. HOFFMANN-LA ROCHE AG, 4070 Basel (CH); Roche Diagnostics GmbH, 68305 Mannheim (DE)
(72) Erfinder: Hunn, Marcel, 4900 Langenthal (CH)
(74) Vertreter: Wess, Wolfgang

(56) Entgegenhaltungen:
- US-A- 5 299 571
- US-A1- 2004 138 543
- US-B1- 6 695 860

## Beschreibung

Die Erfindung betrifft eine Vorrichtung zur in-vivo Platzierung einer Sonde. Die Sonde kann insbesondere eine Messsonde sein zur Ermittlung, vorzugsweise kontinuierlichen Ermittlung, des Werts einer Kenngröße oder mehrerer Kenngrößen, die den Gesundheitszustand eines Organismus charakterisiert oder charakterisieren. Bevorzugt findet die Vorrichtung zu derartigen Messzwecken in Therapien wie beispielsweise der Diabetestherapie Verwendung, in denen der Benutzer sich das jeweilige Medikament, beispielsweise Insulin, selbst verabreicht und auch selbst die Sonde platziert und das Messergebnis auswertet.

Aus der US 6,695,860 B1 ist eine Vorrichtung zur in-vivo Platzierung einer Messsonde bekannt, die einen am Ort der Platzierung auf der Haut verbleibenden ex-vivo Teil und als in-vivo Teil die Messsonde umfasst. Die Messsonde wird mittels einer Einstechnadel im Gewebe platziert. Nach der Platzierung wird die Einstechnadel von der Messsonde getrennt und automatisch in den ex-vivo Teil der Vorrichtung zurückbewegt. In der Vorrichtung ist eine Signaleinrichtung untergebracht, die mit der Messsonde in nicht beschriebener Art und Weise kommuniziert. Das Dokument US 6 695 860 B1 bildet die Basis für die in Anspruch 1 definierte Vorrichtung.

Es ist eine Aufgabe der Erfindung, eine Vorrichtung mit einer in lebendem Gewebe platzierbaren Sonde zu schaffen, welche die Platzierung der Sonde in-vivo und die Kommunikation mit der Sonde auf sichere, aber dennoch einfache und daher preiswerte Weise ermöglicht.

Die Erfindung betrifft eine Vorrichtung zur Platzierung einer Sonde in einem lebenden Gewebe, die ein Gehäuse mit einer auf dem Gewebe platzierbaren Unterseite, die Sonde und eine Signaleinrichtung für die Sonde umfasst. Die Sonde ist im Gebrauch der Vorrichtung ein in-vivo Teil, während das Gehäuse und die Signaleinrichtung am Ort der Platzierung auf dem Gewebe, d. h. ex-vivo, verbleiben. Für den Verbleib auf dem Gewebe ist das Gehäuse mit einem oder mehreren Befestigungsmitteln versehen, mittels dem oder denen es auf dem Gewebe vorzugsweise adhäsiv befestigbar ist. So kann das Gehäuse an seiner Unterseite insbesondere mit einem Klebepad oder einem direkt aufgetragenen Adhäsionsmittel versehen sein. Die Sonde ist für eine Einführung in das Gewebe relativ zu dem Gehäuse über dessen Unterseite hinaus in eine Einführrichtung bewegbar. Die Einführrichtung kann rechtwinklig zu der Unterseite, d. h. zu der Oberseite des Gewebes, weisen. Sie kann stattdessen aber auch unter einer Neigung, vorzugsweise einer konstanten Neigung, zu der Unterseite weisen. Die Signaleinrichtung dient der Übertragung oder Verarbeitung von Signalen von der Sonde oder für die Sonde. Der Signalaustausch kann bidirektional sein, bevorzugt ist er unidirektional und erfolgt von der Sonde zu der Signaleinrichtung. Falls die Sonde für ihren Betrieb mit Energie versorgt werden muss, wird diese Energie vorzugsweise von der Signaleinrichtung zu der Sonde übertragen. Die Signaleinrichtung kann hierfür mit einer Energiequelle ausgestattet sein oder selbst nur der Durchleitung von Energie aus einer anderen, vorzugsweise von dem Gehäuse aufgenommenen Energiequelle dienen.

Nach der Erfindung ist die Sonde mittels der Signaleinrichtung in das Gewebe einführbar. Das Gehäuse lagert die Signaleinrichtung zur Erfüllung dieser weiteren Funktion in die Einführrichtung bis in eine Endposition bewegbar. Die Signalrichtung ist mit der Sonde so gekoppelt, dass sie bei ihrer eigenen Bewegung in die Einführrichtung die Sonde mitnimmt. Wenn die Signaleinrichtung ihre Endposition einnimmt, ist die Sonde bei auf dem Gewebe aufliegendem Gehäuse am vorgesehenen Ort im Gewebe platziert.

Aufgrund der Doppelfunktion der Signaleinrichtung, nämlich als Signal- und Einführeinrichtung, wird ein einfacher Aufbau der Vorrichtung erhalten. Da die Signaleinrichtung die Sonde bei der Bewegung in die Einführrichtung einfach mitnimmt, kann die Sonde während der gesamten Einführbewegung ihre Position relativ zu der Signaleinrichtung beibehalten. Obgleich nicht ausgeschlossen werden soll, dass die Kommunikation zwischen der Sonde und der Signaleinrichtung drahtlos erfolgt oder leitungsgebunden über einem Gleitkontakt, ermöglicht die Erfindung aber vorteilhafterweise, dass die Sonde und die Signaleinrichtung über eine mechanisch nicht unterbrochene durchgehende, feste Signalleitung miteinander verbunden sind, die einerseits fest mit der Signaleinrichtung und andererseits fest mit der Sonde verbunden ist. Dies ermöglicht eine sowohl signaltechnisch besonders sichere und auch mechanisch störungsresistente Signalübertragung. Entsprechendes gilt, falls die Sonde von der oder über die Signaleinrichtung auch mit Energie versorgt wird, für die in diesem Fall bevorzugterweise leitungsgebundene Energieübertragung, wobei die Signalleitung in Zweitfunktion gleichzeitig auch die Energieübertragung besorgen oder für die Energieübertragung eine separate Verbindungsleitung vorgesehen sein kann. Bevorzugt ist die wenigstens eine Signalleitung auf oder in einer die Sonde starr mit der Signaleinrichtung verbindenden Verbindungsstruktur fixiert.

Für die vorteilhaft einfache mechanische Konstruktion der Vorrichtung und auch für die einfache und sichere Signalübertragung wäre es zwar grundsätzlich denkbar, dass die Signaleinrichtung einfach nur lose gegen die Sonde drückend diese in die Einführrichtung mitnimmt. Im Falle der bevorzugt festen Verbindung sind die Sonde und die Signaleinrichtung jedoch in und gegen die Einführrichtung unbeweglich miteinander verbunden. Noch bevorzugter sind sie starr, d. h. relativ zueinander in jeder Hinsicht unbeweglich, miteinander verbunden.

In bevorzugter Ausführung führt das Gehäuse die Signaleinrichtung in einem Führungskontakt längs einer in die Einführrichtung erstreckten Führungsbahn, d. h. das Gehäuse und die Signaleinrichtung oder ein Trägerteil für die Signaleinrichtung bilden ein Kurvengetriebe, vorzugsweise ein Schubgelenk, wobei eines aus Gehäuse einerseits und Signaleinrichtung oder Trägerteil andererseits die Führungsbahn und das andere das Eingriffsglied bilden. Obgleich weniger bevorzugt, wäre alternativ jedoch auch denkbar, dass das Gehäuse und die Signaleinrichtung auf andere Weise miteinander gekoppelt sind. Anstatt der bevorzugt reinen Translationsbewegung, noch bevorzugter der reinen Linearbewegung, könnte die Einführbewegung der Signaleinrichtung beispielsweise auch eine Schwenkbewegung sein und die Signaleinrichtung dementsprechend mittels eines Schwenkmechanismus mit dem Gehäuse gekoppelt sein.

Die Sonde kann als Einstecheinrichtung gebildet sein, die selbst die Gewebeoberfläche, vorzugsweise die menschliche Haut, durchstechen kann. Allerdings müsste sie hierfür entsprechend spitz und knickfest gebildet sein. Bevorzugter umfasst die Vorrichtung jedoch eine Einführhilfe für die Sonde, welche die Sonde bei dem Einführen in das Gewebe schützt. Eine Schutzfunktion kann grundsätzlich zwar bereits dadurch erfüllt werden, dass die Einführhilfe die Sonde bei dem Einführen gegen Biegen oder gar Knicken stabilisiert. Hierfür würde es genügen, wenn die Einführhilfe in Einführrichtung in die Sonde ragen oder neben der Sonde angeordnet wäre und diese in Einführrichtung bis wenigstens nahe zu deren distalen Ende überlappen würde. Vorzugsweise verfügt die Einführhilfe jedoch über eine Hüllstruktur, welche die Sonde zumindest im Wesentlichen und zumindest bis nahe zu ihrem distalen Ende schützend umgibt. Bevorzugt bildet die Hüllstruktur eine Einstechnadel, welche die Sonde in Einführrichtung überragt und an ihrem distalen Ende eine Nadelspitze bildet. Insbesondere kann eine Hüllstruktur die Einstechnadel bilden. Falls eine Einführhilfe vorgesehen ist, kann die Sonde vorteilhafterweise zumindest abschnittsweise flexibel sein, so dass sie im Gebrauch nicht oder doch weniger als eine über ihre gesamte Länge starre Sonde Irritationen verursacht, wenn auf das Gehäuse beispielsweise quer zur Gewebeoberfläche eine Kraft ausgeübt wird. Die Einführhilfe wird nach dem Einführen der Sonde wieder aus dem Gewebe gezogen, d. h. sie ist insbesondere von der Sonde oder zumindest einem in das Gewebe ragenden Teil der Sonde trennbar.

Für das Zusammenwirken mit einer Einführhilfe ist es vorteilhaft, wenn die Sonde ein freies proximales Ende und von dem proximalen Ende bis wenigstens nahezu ihrem distalen Ende durchgehend eine freie, d. h. zugängliche Oberfläche aufweist und so eine schützende, vorzugsweise stabilisierende Überlappung durch die Einführhilfe ermöglicht. Für die Stabilisierung sollte die Einführhilfe die Sonde in der Überlappung kontaktieren. Bevorzugt kontaktieren die Sonde und die Einführhilfe einander flächig über die freie Oberfläche der Sonde.

Falls für die Einführung der Sonde eine Einstechnadel vorgesehen ist, bildet eine derartige Einführhilfe vorzugsweise einen Nadelschutz, um nach der Trennung von der Sonde den Benutzer gegen Stichverletzungen durch die Einstechnadel zu schützen. In bevorzugter Ausführung umfasst die Einführhilfe hierfür zwei Schwenkflügel, die aus einem ausgeschwenkten Zustand, den die Einführhilfe für die Einführung der Sonde einnimmt, in einen Schutzzustand aufeinander zu schwenkbar sind. Im Schutzzustand liegen die Schwenkflügel im Wesentlichen längs der Einstechnadel und schirmen deren Spitze nach außen ab. Die Schwenkflügel können stabförmig sein. Vorzugsweise sind sie plättchenförmig oder gewölbt, um die Nadelspitze sicher nicht nur gegen einen Zugriff von vorne, sondern auch gegen seitlichen Zugriff abzuschirmen.

Die Signaleinrichtung ist auf einem Trägerteil gelagert, vorzugsweise ist sie in dem Trägerteil eingebettet. Das Gehäuse lagert das Trägerteil in die Einführrichtung bis in die Endposition bewegbar, vorzugsweise im Gleitkontakt bewegbar. In bevorzugten Ausführungen ist die Vorrichtung zweiteilig und besteht aus dem der Platzierung und vorzugsweise Fixierung auf der Gewebeoberfläche dienenden Gehäuse und dem die Signaleinrichtung tragenden Trägerteil. Für die Einführung der Sonde ist vorzugsweise die Einführhilfe vorgesehen, die in derartigen Ausführungen noch als ein drittes Teil hinzukommt, aber nach dem Platzieren der Sonde entfernt wird, so dass auch in derartigen Ausführungen im Betrieb der Sonde die Vorrichtung nur zweiteilig ist, d. h. nur zwei relativ zueinander bewegbare Teile umfasst. Das Gehäuse und das Trägerteil bilden allerdings eine Einheit in dem Sinne, dass sie im Gebrauch, insbesondere bei der Platzierung der Sonde und in deren Betrieb nach der Platzierung von der für die Platzierung erforderlichen Bewegbarkeit des Trägerteils relativ zu dem Gehäuse abgesehen fest miteinander verbunden, insbesondere als Einheit handhabbar sind, so dass durch die Platzierung des Gehäuses auf der Gewebeoberfläche auch gleichzeitig das Trägerteil platziert ist und lediglich noch aus einer Ausgangsposition relativ zu dem Gehäuse in die Endposition bewegt werden muss. Das Trägerteil ist in der Ausgangsposition an dem Gehäuse vorteilhafterweise fixiert. Die Fixierung ist allerdings lösbar, damit das Trägerteil in die Einführrichtung bewegt werden kann. Vorzugsweise löst sich die Fixierung automatisch bei Ausübung einer in die Einführrichtung auf das Trägerteil wirkenden Kraft. Die Fixierung kann beispielsweise form- und kraftschlüssig durch einen lösbaren Rasteingriff, oder über Reibflächen rein reibschlüssig oder auch stoffschlüssig hergestellt werden, wobei im letzteren Fall der oder die den Stoffschluss herstellenden Verbindungssteg(e) durch Ausübung einer in die Einführrichtung auf das Trägerteil wirkenden Kraft zerstört wird oder werden. Das Trägerteil ist vorzugsweise auch in der Endposition an dem Gehäuse fixiert, vorteilhafterweise fixiert es sich bei Erreichen der Endposition automatisch an dem Gehäuse. Die Fixierung in der Endposition kann lösbar oder unlösbar sein. Sie kann insbesondere form- und kraftschlüssig durch Verrastung des Trägerteils mit dem Gehäuse oder auch wieder rein reibschlüssig gebildet sein. Die Fixierung in der Endposition ist vorteilhafterweise wenigstens so fest, dass sich das Trägerteil im Gebrauch nicht versehentlich relativ zu dem Gehäuse gegen die Einführrichtung bewegen kann.

Das Trägerteil hält vorzugsweise auch die Sonde und bildet in derartigen Ausführungen somit auch einen Sondenhalter. Vorteilhafterweise ist die Sonde mit dem Trägerteil so verbunden, dass sie von einer von der Unterseite des Gehäuses abgewandten Seite, vorzugsweise von einer der Unterseite des Gehäuses gegenüberliegenden Oberseite des Trägerteils, in Einführrichtung zugänglich ist. Diese Zugänglichkeit ist insbesondere dann von Vorteil, wenn die Vorrichtung die genannte Einführhilfe umfasst, da die Einführhilfe dann sehr einfach gegen die Einführrichtung von der Sonde und dem Trägerteil abgezogen werden kann. Die Sonde kann hierfür an einer Seitenwand an einem äußeren Umfang des Trägerteils oder in einer Ausnehmung oder Durchbrechung des Trägerteils angeordnet und am Trägerteil befestigt sein. Besonders bevorzugt ist das Trägerteil hierfür mit einer in die Einführrichtung weisenden, bevorzugt zentralen Durchbrechung versehen. Die Sonde erstreckt sich vorzugsweise aus einem in Bezug auf den Querschnitt zentralen Bereich der Durchbrechung über das Trägerteil hinaus in die Einführrichtung.

Die Sonde kann grundsätzlich zwar bereits in der Ausgangsposition des Trägerteils über die Unterseite des Gehäuses vorstehen, vorzugsweise steht sie hinter der Unterseite jedoch zurück, so dass sie von einem die Unterseite bildenden Boden des Gehäuses geschützt wird. Falls die Vorrichtung über eine Einführhilfe verfügt, kann diese in einem Lagerungszustand, in dem die Vorrichtung vor einem Gebrauch gelagert werden kann, auf der von der Einführrichtung abgewandten Seite der Vorrichtung einen weiteren Schutz für die Sonde bilden. Die Sonde kann so in einem für die Bewegbarkeit des Trägerteils zwischen dem Gehäuse und dem Trägerteil gebildeten Hohlraum aufgenommen, vorzugsweise eingekapselt sein.

Die Sonde kann für die Platzierung in muskulärem Gewebe oder beispielsweise einer Vene vorgesehen sein. Vorzugsweise ist sie für die Platzierung unter der Haut in hautnahem Gewebe, d. h. subkutan, oder gegebenenfalls in der Haut vorgesehen. Sie kann der Zuführung eines zu verabreichenden Produktfluids dienen und in solch einer Ausführung einen Katheterkopf bilden, der über einen Katheter mit einem Infusionsgerät verbunden ist. In bevorzugter Ausführung ist die Sonde als Messsonde ausgeführt und wird zur Ermittlung, vorzugsweise zur kontinuierlichen Ermittlung über mehrere Stunden oder Tage, einer den Gesundheitszustand des Organismus kennzeichnenden Kenngröße, vorzugsweise einer biochemischen Kenngröße, verwendet. Solch eine Messsonde kann nach einem auf Stoffaustausch beruhenden Verfahren arbeiten und in derartigen Ausführungen beispielsweise als enzymatischer Sensor oder viskometrischer Affinitätssensor gebildet sein. Enzymatische Sensoren und insbesondere viskometrische Affinitätssensoren eignen sich im besonderen Maße für die Bestimmung der Glucosekonzentration. Die Messsonde kann in alternativen Ausführungen aber auch nach anderen Verfahren ohne Stofftransport in oder durch die oder aus der Sonde arbeiten, beispielsweise als Infrarotsonde. Die explizit genannten Sondentypen sollen nur als Beispiele verstanden werden, grundsätzlich kann die Sonde beispielsweise ein reiner Temperatursensor sein oder beispielsweise Elektroden für eine Widerstandsmessung umfassen. Die Sonde kann auch mit einer Kombination aus mehreren Sensortypen, insbesondere mehreren der genannten Sensortypen ausgestattet sein. Sie wird in bevorzugter Verwendung zur kontinuierlichen Überwachung der einen oder der mehreren Kenngrößen verwendet. Schließlich kann sie auch eine Messsonde und eine Sonde zur Verabreichung eines Produktfluids in Kombination sein, wobei mittels des Messteils einer solchen Kombinationssonde vorteilhafterweise eine für die Verabreichung des Produktfluids maßgebliche Kenngröße ermittelt wird, um die Zuführrate des Produktfluids in einen geschlossenen Regelkreis regeln oder zumindest anhand der ermittelten Werte durch Benutzereingriff an einem Verabreichungsgerät steuern zu können.

Die Signaleinrichtung kann als reine Durchleiteinrichtung gebildet sein, die von der Sonde erhaltene Signale zu einer Verarbeitungseinheit, beispielsweise einem vom Benutzer getragenen Infusionsgerät oder einem Handheld-Computer bzw. Palm, PC, Laptop oder vergleichbaren Datenendgerät weiterleitet. Um eine drahtlose Weiterleitung zu ermöglichen, ist eine derartige Signaleinrichtung mit einem Sender, vorzugsweise einem Funksender, ausgestattet. Um die erhaltenen Signale weiterzuleiten, kann die Signaleinrichtung auch über einen Signalspeicher, vorzugsweise digitalen Datenspeicher, verfügen. Falls ein Speicher vorhanden ist, kann auf eine drahtlose oder drahtgebundene Weiterleitung der Signale zu einer Verarbeitungseinrichtung grundsätzlich sogar verzichtet werden, falls es nämlich auf eine zeitnahe Auswertung der Signale nicht ankommt. In solch einem Fall kann nach Gebrauch der Vorrichtung die Signaleinrichtung oder nur deren Speicher mit der Verarbeitungseinrichtung zu Zwecken der Auswertung verbunden werden, vorzugsweise über einen Standardanschluss, etwa ein USB-Anschluss. In bevorzugten Ausführungen bildet die Signaleinrichtung jedoch selbst eine Verarbeitungseinrichtung für die erhaltenen Signale. Die Signaleinrichtung ist vorzugsweise auch in derartigen Ausführungen zur Kommunikation mit einer weiteren Verarbeitungseinrichtung fähig, die bevorzugt ein Verabreichungsgerät bildet, beispielsweise eine Infusionspumpe, oder ein Handheld-Computer bzw. Palm, ein PC oder ein Laptop. Die Signaleinrichtung kommuniziert in derartigen Ausführungen ebenfalls vorzugsweise drahtlos, bevorzugt per Funk, oder gegebenenfalls drahtgebunden mit der weiteren Verarbeitungseinrichtung. Die Vorrichtung kann mit einer optischen und/oder akustischen und/oder taktilen Anzeige ausgestattet sein, um die Messwerte oder daraus von der Signaleinrichtung abgeleitete Kenngrößenwert anzuzeigen, beispielsweise auch nur mittels eines akustischen und/oder taktilen Alarmsignals, beispielsweise einem Vibrationssignal.

Die Vorrichtung wird besonders bevorzugt in Kombination mit einem Verabreichungsgerät verwendet, das der Benutzer der Vorrichtung für die Verabreichung ständig oder zumindest über längere Zeiträume am Körper trägt. Das Verabreichungsgerät und die Vorrichtung sind für die Kommunikation aufeinander abgestimmt. Die Kommunikation kann bidirektional sein, in bevorzugt einfachen Ausführungen ist sie unidirektional, wobei das Verabreichungsgerät Signale von der Vorrichtung erhält, vorzugsweise per Funk. Die Signale, vorzugsweise bereits von der Signaleinrichtung aufbereitete Messwerte oder gegebenenfalls daraus bereits abgeleitete Kenngrößenwerte, werden zu dem Verabreichungsgerät übertragen und von dessen Verarbeitungseinrichtung, soweit erforderlich, weiterverarbeitet. Die Messwerte oder die daraus abgeleiteten Kenngrößenwerte werden an dem Verabreichungsgerät zur Anzeige gebracht, vorzugsweise optisch oder im Falle des Feststellens kritischer Zustände auch in Form eines akustischen und/oder taktilen Alarmsignals. Das Verabreichungsgerät kann die Messwerte oder die daraus abgeleiteten Kenngrößenwerte ständig oder nur auf Verlangen des Benutzers zur Anzeige bringen. Die Vorrichtung kann auch mit einem speziell abgestimmten, separaten Anzeigegerät angeboten werden, das bequem in der Tasche tragbar ist und vorzugsweise über Funk mit der Signaleinrichtung kommuniziert. Das Anzeigegerät verfügt vorzugsweise über eine eigene Verarbeitungseinrichtung zur Verarbeitung der von der Signaleinrichtung erhaltenen Signale. Das separate Anzeigegerät kann insbesondere ein handelsüblicher Handheld-Computer bzw. Palm oder ein vergleichbares Gerät sein, das über eine Standard-Schnittstelle, beispielsweise Bluetooth, mit der Signaleinrichtung kommuniziert. Die Signaleinrichtung ist mit einer entsprechenden Standard-Schnittstelle, beispielsweise einem Bluetooth-Sender und/oder -empfänger ausgerüstet. Falls die Vorrichtung in Kombination mit einem Verabreichungsgerät angeboten wird, gilt vorteilhafterweise entsprechendes.

Was die Energieversorgung anbetrifft, ist die Vorrichtung vorzugsweise autark, d. h. sie ist selbst mit einer Energiequelle, vorzugsweise einer Batterie, ausgestattet. Das genannte Trägerteil lagert vorteilhafterweise auch die Energiequelle.

Vorteilhafte Merkmale werden auch in den Unteransprüchen und deren Kombinationen beschrieben. Die durch die Unteransprüche offenbarten Merkmale bilden die vorstehend beschriebenen Ausgestaltungen weiter oder wandeln sie ab. Ebenso werden die durch die Unteransprüche offenbarten Ausgestaltungen durch die vorstehend beschriebenen weitergebildet oder abgewandelt.

Ausführungsbeispiele der Erfindung werden nachfolgend anhand von Figuren erläutert. An den Figuren offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: eine Vorrichtung für die Platzierung einer Sonde in lebendem Gewebe,
- Figur 2: die Sonde und eine die Sonde schützende Hüllstruktur und
- Figuren 3-6: die Platzierung der Sonde in dem Gewebe im Ablauf.

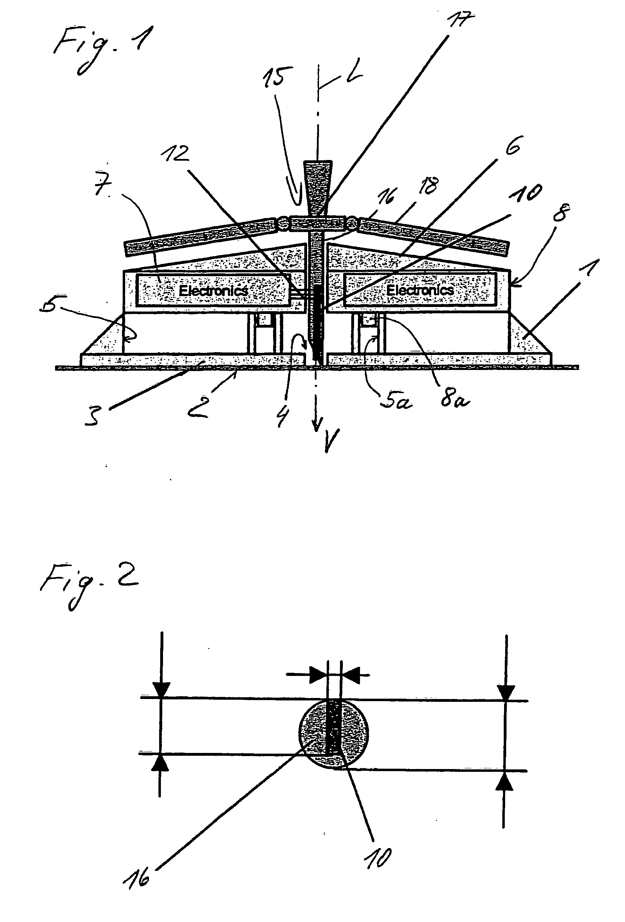

Figur 1 zeigt eine Vorrichtung für die Platzierung einer Sonde in lebendem Gewebe, vorzugsweise im menschlichen Körper, in einem Längsschnitt. Die Vorrichtung umfasst ein Gehäuse 1 mit einer Unterseite 2 zur Platzierung und Befestigung der Vorrichtung auf der Gewebeoberfläche, vorzugsweise der Haut. Ferner umfasst die Vorrichtung eine Signaleinrichtung 7 und ein Trägerteil 6, das sowohl die Signaleinrichtung 7 als auch die Sonde 10 trägt. Die Signaleinrichtung 7 ist in das Trägerteil 6 eingebettet, und die Sonde 10 ist mit dem Trägerteil 5 starr verbunden. Die Sonde 10 ist über die starre Verbindung mittels mehrerer Signalleitungen 12 signaltechnisch mit der Signaleinrichtung 7 verbunden. Die Sonde 10 ist als Messsonde für die subkutane Platzierung und Messung einer den Gesundheitszustand eines Organismus, vorzugsweise des Menschen, charakterisierenden Kenngröße gebildet. Die Vorrichtung des Ausführungsbeispiels dient der kontinuierlichen Überwachung der Glucosekonzentration im subkutanen Gewebe. Die Sonde 10 bildet dementsprechend einen Glucosesensor.

Die Signaleinrichtung 7 ist als elektronische Signalverarbeitungseinrichtung gebildet. Sie empfängt über die Signalleitungen 12 Messsignale von der Sonde 10, vorzugsweise elektronische Messsignale. In alternativen Ausführungen können die Signalleitungen 12 auch durch eine Stoffzuleitung und eine Stoffableitung zu und von der Sonde 10 gebildet sein, falls die Sonde beispielsweise als viskosimetrischer Affinitätssensor gebildet ist und die Signaleinrichtung 7 eine durch die Sonde 10 geleitete, mit Glucose angereicherte Flüssigkeit in an sich bekannter Weise auswertet. Falls die Sonde 10 als viskosimetrischer Affinitätssensor gebildet ist, findet die Ableitung von Signalen jedoch vorzugsweise noch in der Sonde 10 statt, und es werden die Messsignale der Sonde 10 elektronisch oder gegebenenfalls optisch über die Signalleitungen 12 zur Signaleinrichtung 7 übertragen.

Das Trägerteil 6 nimmt in dem in Figur 1 gezeigten Lagerungszustand der Vorrichtung relativ zu dem Gehäuse 1 eine Ausgangsposition ein und ist in der Ausgangsposition mit dem Gehäuse 1 lösbar verrastet. Die Sonde 10 ist stabförmig mit einer Längsachse L. Das Trägerteil 6 ist relativ zu dem Gehäuse 1 entlang der Längsachse L in eine Einführrichtung V aus der in Figur 1 eingenommenen Ausgangsposition bis in eine Endposition linear bewegbar. Während der Bewegung führt das Gehäuse 1 das Trägerteil 6 in einem Führungskontakt. Der Führungskontakt ist als Gleitkontakt einer Führungsbahn 5 des Gehäuses 1 und einer Führungsbahn 8 des Trägerteils 6 gebildet. Eine Mantelinnenfläche des Gehäuses 1 bildet die Führungsbahn 5 und eine Mantelaußenfläche des Trägerteils 6 bildet die Führungsbahn 8. Das Trägerteil 6 ist mit dem Gehäuse 1 ferner verdrehgesichert verbunden, wofür das Gehäuse 1 mit Eingriffselementen 5a und das Trägerteil 6 mit Eingriffselementen 8a versehen sind.

Das Gehäuse 1 weist einen scheibenförmigen, flachen Boden 3 auf, an dessen Unterseite ein Klebepad angebracht ist, das die Unterseite 2 des Gehäuses 1 bildet. Anstatt eines zusätzlichen Klebepads könnte auch unmittelbar der Boden 3 die Unterseite des Gehäuses 1 bilden und hierfür beispielsweise selbst mit einem Adhäsionsmittel versehen sein. Der Boden 3 weist eine zentrale Durchbrechung 4 auf. Von dem Boden 3 ragt an dessen von der Unterseite 2 abgewandten Oberseite um die Durchbrechung 4 umlaufend, vorzugsweise zylindrisch umlaufend, eine Gehäusewandung mit einer zu der Einführrichtung V parallelen Mantelinnenfläche auf, welche die Führungsbahn 5 des Gehäuses 1 bildet. Die Führungsbahn 8 des Trägerteils 6 ist dementsprechend ebenfalls zu der Einführrichtung V parallel. Das Gehäuse 1 und das Trägerteil 6 bilden ein Schubgelenk mit den Führungsbahnen 5 und 8 als Führungskurve und Eingriffsglied. Die Führungsbahnen 5 und 8 erstrecken sich jeweils über die gesamte Länge der Bewegbarkeit des Trägerteils 6. Grundsätzlich würde es jedoch genügen, wenn sich nur eine der Führungsbahnen 5 und 8 über die gesamte Länge und die andere nur über einen Längenabschnitt erstrecken würde.

Die Sonde 10 ist an dem Trägerteil 6 so angebracht, dass ihre Längsachse L in Einführrichtung V weist. Das Trägerteil 6 ist längs der Längsachse L in der Flucht zu der Durchbrechung 4 ebenfalls mit einer Durchbrechung versehen, in der die Sonde 10 in die Einführrichtung V weisend befestigt ist. Die Sonde 10 ist in der Durchbrechung des Trägerteils 6 bezüglich des Querschnitts der Durchbrechung zentral angeordnet und mittels eines seitlichen Verbindungsstegs mit der Trägerstruktur 6 mechanisch verbunden. Die Signalleitungen 12 sind in oder auf dem Verbindungssteg fixiert. Die Sonde 10 und die Signaleinrichtung 7 sind auf diese Weise steif miteinander verbunden. Insbesondere besteht permanent über die Signalleitungen 12 eine sich nicht verändernde signaltechnische Verbindung.

In dem in Figur 1 gezeigten Lagerungszustand umschließen das Gehäuse 1 und das Trägerteil 6 einen Hohlraum. Die Sonde 10 ragt aus der Durchbrechung des Trägerteils 6 in den Hohlraum, wobei ihr distales Ende der Durchbrechung 4 des Gehäuses 1 gegenüberliegt, wobei die Sonde 10 mit ihrem distalen Ende sogar in die Durchbrechung 4 ragt. Im Lagerungszustand wird die Durchbrechung 4 allerdings von dem Klebepad verschlossen.

Die Vorrichtung umfasst im Lagerungszustand ferner eine Einführhilfe 15, welche die Sonde 10 insbesondere bei dem Einführen in das Gewebe schützt. Der Schutz besteht im Wesentlichen in einer Stabilisierung der Sonde 10 gegen ein Biegen oder gar Knicken. Die Einführhilfe 15 umfasst eine Hüllstruktur 16, die im Lagerungszustand der Vorrichtung die Durchbrechung des Trägerteils 6 durchragend die Sonde 10 umgibt. Die Hüllstruktur 16 ist als Einstechnadel gebildet und wird im Folgenden so bezeichnet. Sie überragt die Sonde 10 in die Einführrichtung V, steht aber ebenfalls noch hinter dem Klebepad zurück. Das distale Ende der Einstechnadel 16 ist zugespitzt. Die Einführhilfe 15 umfasst des Weiteren einen Nadelhalter 17, von dem die Einstechnadel 16 abragt, und beidseits der Einstechnadel 16 je einen Schwenkflügel 18. Die Schwenkflügel 18 sind mittels je eines Schwenkgelenks mit dem Nadelhalter 17 verbunden. Sie sind aus einer jeweils abgeschwenkten Position, die sie im Lagerungszustand der Vorrichtung einnehmen, auf die Einstechnadel 16 zu in eine Schutzposition schwenkbar. An ihrer von der Einstechnadel 16 abgewandten Seite weist die Einführhilfe 15 einen Griff auf, an dem ein Benutzer der Vorrichtung die Einführhilfe 15 greifen und gegen die Einführrichtung V von der Sonde 10 und dem Trägerteil 6 abziehen kann.

Figur 2 zeigt die Sonde 10 und die Einstechnadel 16 in einem Querschnitt. Die Einstechnadel 16 umgibt die Sonde 10 eng anliegend über deren nahezu gesamte freie Oberfläche. Sie weist hierfür einen vollen Querschnitt auf, der mit einem Aufnahmeschlitz für die Sonde 10 versehen ist. An einer Seite, an der die Sonde quer zu der Einführrichtung V mit dem Trägerteil 6 verbunden ist, mündet der Schlitz an der äußeren Umfangsfläche der Einstechnadel 16. Um die Einstechnadel 16 nach der in-vivo Platzierung der Sonde 10 von der Sonde 10 abziehen zu können, erstreckt sich der Schlitz bis zu dem distalen Ende der Einstechnadel 16.

Die Sonde 10 ragt über das Trägerteil 6 in die Einführrichtung V mit einer Länge hinaus, die für eine subkutane Platzierung geeignet ist. Die über das Trägerteil 6 hinausragende Länge sollte wenigstens 4 mm betragen und vorzugsweise 12 mm nicht übersteigen. Entsprechend lang ist wenigstens eine der Führungsbahnen 5 und 8 und entsprechend hoch der zwischen dem Gehäuseboden 3 und dem Trägerteil 6 verbleibende Hohlraum. In der Endposition kontaktiert das Trägerteil 6 vorzugsweise direkt den Gehäuseboden 3. Vorzugsweise liegt es in der Endposition auf dem Boden 3 flächig auf. In der Endposition ist das Trägerteil 6 an dem Gehäuse 1 vorteilhafterweise ausreichend fest fixiert, so dass unter den im üblichen Gebrauch zu erwartenden Belastungen das Trägerteil 6 die Endposition nicht verlassen kann. Die Fixierung kann beispielsweise als Rasteingriff von Gehäuse 1 und Trägerteil 6 oder Reibschluss zwischen den Führungsbahnen 5 und 8 gebildet sein. Um eine rechtwinklig auf die Unterseite gemessen möglichst flache Vorrichtung zu erhalten, sollte der Gehäuseboden 3 möglichst dünn sein, was auch vorteilhaft ist, um die Unterseite des Gehäuses 1 mit einer gewissen Flexibilität auszustatten, so dass sie nicht völlig starr ist, sondern sich einer Wölbung der Gewebeoberfläche anpassen kann. Einer flachen Bauweise kommt es ferner entgegen, wenn das Gehäuse 1 von der Oberseite des Bodens 3 rechtwinklig zur Unterseite 2 nur so weit aufragt, wie zur Führung des Trägerteils 6 und dessen Fixierung in der Ausgangsposition erforderlich. Entsprechendes gilt auch für das Trägerteil 6. Falls die Einführrichtung V wie im Ausführungsbeispiels rechtwinklig zu der Unterseite 2 weist, kann die Vorrichtung bei in der Endposition befindlichem Trägerteil 6 nach dem Entfernen der Einführhilfe 15 eine rechtwinklig auf die Unterseite 2 gemessene Höhe über alles haben, die in etwa der Länge entspricht, mit der die Sonde 10 in die Einführrichtung V über das Lagerteil 6 hinaus ragt. Der flachen Bauweise kommt auch entgegen, dass die Einführrichtung V rechtwinklig zu der Unterseite 2 weist.

Nachfolgend wird die Handhabung der Vorrichtung bei der in-vivo Platzierung der Sonde 10 anhand der Figuren 1 und 3-6 beschrieben:
Der Benutzer erhält die Vorrichtung in dem in Figur 1 gezeigten Lagerungszustand. Die Sonde 10 ist in dem zwischen dem Gehäuse 1 und dem Trägerteil 6 gebildeten Hohlraum steril aufgenommen. Das Trägerteil 6 nimmt seine Ausgangsposition ein. Die Einführhilfe 17 liegt auf der Oberseite des Trägerteils 6 auf oder dieser Oberseite geringfügig beabstandet gegenüber, wie in Figur 1 dargestellt. Im Lagerungszustand nimmt der Benutzer mit der einen Hand die Vorrichtung und entfernt eine Abdeckung an der Unterseite 2 des Klebepads. Anschließend setzt er die Vorrichtung mit der freigelegten Klebefläche des Klebepads über dem gewünschten Messort auf die Hautoberfläche auf. Die Vorrichtung ist nun mit ihrer Unterseite 2 auf der Hautoberfläche adhäsiv fixiert. Im nächsten Schritt drückt er das Trägerteil 6 in die Einführrichtung V bis gegen den Boden 3, d. h. in die Endposition relativ zu dem Gehäuse 1. Die Einstechnadel 16 durchsticht die Haut und dringt bis in das darunter liegende, hautnahe Gewebe ein. Die Einführbewegung wird durch eine manuell aufgebrachte, auf das Trägerteil 6 in die Einführrichtung V wirkende Druckkraft bewirkt. Der Benutzer übt die Druckkraft mittels der Einführhilfe 15, d. h. mittels des Griffs der Einführhilfe 15, aus. Aufgrund der festen Verbindung zwischen dem Trägerteil 6 und der Sonde 10 nimmt das Trägerteil 6 bei seiner Einführbewegung die Sonde 10 mit. Während des Durchstechens der Haut und des weiteren Einführens in das tiefer gelegene Gewebe schützt die Einstechnadel 16 die Sonde 10.

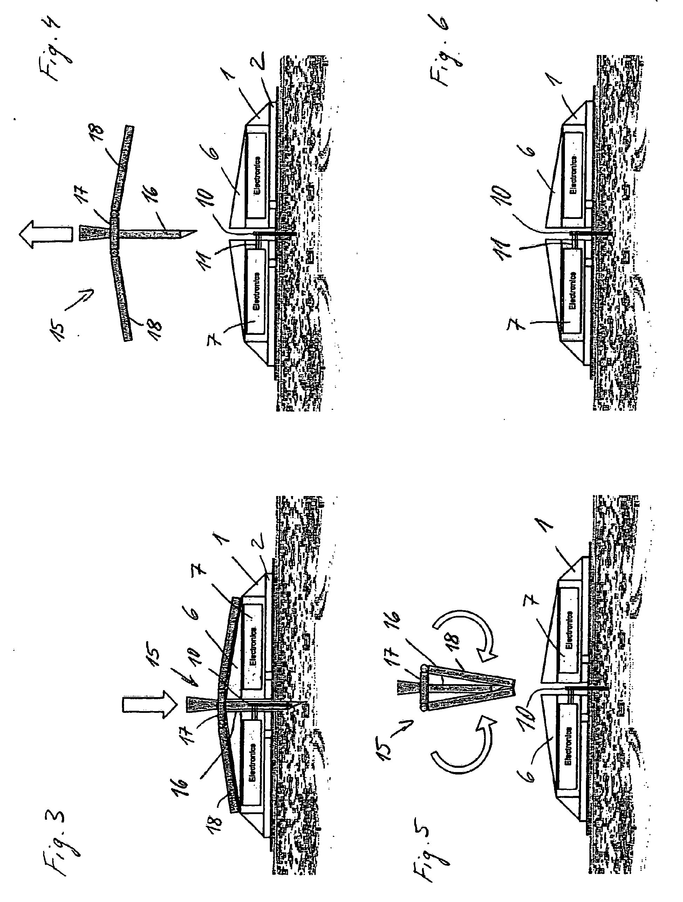

Figur 3 zeigt die Vorrichtung nach dem vollständigen Einführen der Sonde 10, d. h. das Trägerteil 6 nimmt die Endposition ein. Die Einführhilfe 15 liegt noch auf der Oberseite des Trägerteils 6 auf.
Figur 4 zeigt die Vorrichtung, nachdem die Einführhilfe 15 von der Sonde 10 und auch bereits von dem Trägerteil 6 getrennt wurde. Für das Trennen greift der Benutzer die Einführhilfe 15 am Griff und zieht sie gegen die Einführrichtung V gerade von der Sonde 10 ab und aus der Durchbrechung des Trägerteils 6 heraus.

Für die sichere Handhabung, beispielsweise Entsorgung, der Einführhilfe 15 schwenkt der Benutzer die Schwenkflügel 18 in Richtung auf die Einstechnadel 16 zu bis in den in Figur 5 gezeigten Schutzzustand. Im Schutzzustand können die Schwenkflügel 18 vorteilhafterweise je eine Rastposition einnehmen oder miteinander verrasten, so dass sie die Schutzposition nicht versehentlich verlassen können. Alternativ oder zusätzlich können die beiden Schwenkgelenke auch entsprechend schwergängig gestaltet sein.

In Figur 6 schließlich ist die Vorrichtung im Betriebszustand gezeigt. Die Sonde 10 gibt an die Signaleinrichtung 7 kontinuierlich Messsignale aus, aus denen die aktuelle Glucosekonzentration im Gewebe ermittelbar ist.

### Bezugszeichen:

- 1: Gehäuse
- 2: Unterseite
- 3: Boden
- 4: Durchbrechung
- 5: Führungsbahn, Eingriffsglied
- 5a: Eingriffselement, Verdrehsicherung
- 6: Trägerteil
- 7: Signaleinrichtung
- 8: Eingriffsglied, Führungsbahn
- 8a: Eingriffselement Verdrehsicherung
- 9: -
- 10: Sonde
- 11: -
- 12: Signalleitung
- 13: -
- 14: -
- 15: Einführhilfe
- 16: Hüllstruktur, Einstechnadel
- 17: Nadelhalter
- 18: Schwenkflügel

- L: Längsachse
- V: Einführrichtung

## Patentansprüche

1. Vorrichtung zur Platzierung einer Sonde in lebendem Gewebe, die Vorrichtung umfassend:
a) ein Gehäuse (1) mit einer Unterseite (2) für die Platzierung auf dem Gewebe,
b) die für eine Einführung in das Gewebe relativ zu dem Gehäuse (1) über die Unterseite (2) hinaus in eine Einführrichtung (V) bewegbare Sonde (10)
c) und eine Signaleinrichtung (7) für die Sonde (10), **dadurch gekennzeichnet, daß**
d) die Signaleinrichtung (7) von dem Gehäuse (1) in die Einführrichtung (V) bis in eine Endposition bewegbar gelagert ist und bei einer Bewegung in die Einführrichtung (V) die Sonde (10) mitnimmt.

2. Vorrichtung nach Anspruch 1, **dadurch gekennzeichnet, dass** die Sonde (10) und die Signaleinrichtung (7) mittels wenigstens einer Signalleitung (12) miteinander verbunden sind.

3. Vorrichtung nach einem der vorhergehenden Ansprüche, **dadurch gekennzeichnet, dass** die Sonde (10) und die Signaleinrichtung (7) in und gegen die Einführrichtung (V) unbeweglich miteinander verbunden sind.

4. Vorrichtung nach einem der vorhergehenden Ansprüche, **dadurch gekennzeichnet, dass** das Gehäuse (1) die Signaleinrichtung (7) in einem Führungskontakt längs einer in die Einführrichtung (V) erstreckten Führungsbahn (5, 8) führt.

5. Vorrichtung nach einem der vorhergehenden Ansprüche, **dadurch gekennzeichnet, dass** die Sonde (10) ein freies proximales Ende und ein freies distales Ende und eine von dem proximalen Ende bis wenigstens nahe zu dem distalen Ende in Einführrichtung (V) durchgehend freie Oberfläche aufweist.

6. Vorrichtung nach einem der vorhergehenden Ansprüche, **dadurch gekennzeichnet, dass** die Sonde (10) über einen seitlich von der Sonde (10) abragenden Verbindungssteg mit der Signaleinrichtung (7) verbunden ist.

7. Vorrichtung nach einem der vorhergehenden Ansprüche, ferner umfassend ein die Signaleinrichtung (7) haltendes Trägerteil (6), das in Einführrichtung (V) eine Durchbrechung aufweist, aus der die Sonde (10) in die Einführrichtung (V) hinausragt.

8. Vorrichtung nach dem vorhergehenden Anspruch, **dadurch gekennzeichnet, dass** die Sonde (10) an dem Trägerteil (6) befestigt ist.

9. Vorrichtung nach einem der zwei vorhergehenden Ansprüche 7, 8, **dadurch gekennzeichnet, dass** die Sonde (10) das Trägerteil (6) in Einführrichtung (V) in einem Sondenabschnitt überlappt und im Bereich des überlappenden Sondenabschnitts mit dem Trägerteil (6) in eine Richtung quer zu der Einführrichtung (V) verbunden ist.

10. Vorrichtung nach einem der drei vorhergehenden Ansprüche 7, 8, 9, **dadurch gekennzeichnet, dass** das Trägerteil (6) in einem Gleitkontakt mit dem Gehäuse (1) in die Einführrichtung (V) bewegbar geführt wird.

11. Vorrichtung nach dem vorhergehenden Anspruch, **dadurch gekennzeichnet, dass** das Trägerteil (6) und das Gehäuse (1) an einander quer zu der Einführrichtung (V) zugewandten Umfangsflächen in dem Gleitkontakt befindliche Führungsbahnen (5, 8) bilden.

12. Vorrichtung nach einem der vorhergehenden Ansprüche, ferner umfassend eine Einführhilfe (15) für die Sonde (10), welche die Sonde (10) in Einführrichtung (V) wenigstens über einen wesentlichen Teil ihrer Länge überlappt und die gegen die Einführrichtung (V) von der Sonde (10) abziehbar ist.

13. Vorrichtung nach dem vorhergehenden Anspruch, **dadurch gekennzeichnet, dass** die Einführhilfe (15) eine Hüllstruktur (16) umfasst, welche die Sonde (10) über wenigstens einen größeren Teil eines Umfangs der Sonde (10) umgibt.

14. Vorrichtung nach dem vorhergehenden Anspruch, **dadurch gekennzeichnet, dass** die Hüllstruktur (16) einen in Einführrichtung (V) erstreckten Schlitz aufweist, in dem die Sonde (10) aufgenommen ist, und dass der Schlitz über die gesamte Länge der Sonde (10) an einer äußeren Umfangsfläche der Hüllstruktur (16) mündet.

15. Vorrichtung nach einem der drei vorhergehenden Ansprüche 12, 13, 14, **dadurch gekennzeichnet, dass** die Einführhilfe (15) eine Einstechnadel (16) umfasst, welche die Sonde (10) in Einführrichtung (V) überragt, und dass die Sonde (10) an der oder um die oder vorzugsweise in der Einstechnadel (16) angeordnet ist.

16. Vorrichtung nach dem vorhergehenden Anspruch, **dadurch gekennzeichnet, dass** die Einstechhilfe (15) wenigstens zwei Schwenkflügel (18) umfasst, die aufeinander zu in Schutzposition schwenkbar sind, so dass die Einstechnadel (16) zwischen den Schwenkflügeln (18) zu liegen kommt, und dass die Schwenkflügel (18) in der Schutzposition über eine Spitze der Einstechnadel (16) hinausragen.

17. Vorrichtung nach einem der vorhergehenden Ansprüche, **dadurch gekennzeichnet, dass** die Sonde (10) eine Messsonde zur Aufnahme einer den Gesundheitszustand eines Organismus charakterisierenden Kenngröße, vorzugsweise biochemischen Kenngröße (Glucosekonzentration) ist.

18. Vorrichtung nach einem der vorhergehenden Ansprüche, **gekennzeichnet durch** die Verwendung zur Ermittlung, vorzugsweise kontinuierlichen Ermittlung, einer den Gesundheitszustand eines Organismus charakterisierenden Kenngröße, vorzugsweise biochemischen Kenngröße (Glucosekonzentration).

## Claims

1. A device for positioning a probe in living tissue, said device comprising:
a) a casing (1) having an underside (2) for positioning on the tissue;
b) the probe (10), which can be moved relative to the casing (1), beyond the underside (2) in an insertion direction (V), in order to be inserted into the tissue;
c) and a signal means (7) for the probe (10),
**characterised in that**
d) the signal means (7) is mounted by the casing (1) such that it can be moved in the insertion direction (V) up to an end position, and slaves the probe (10) when moving in the insertion direction (V).

2. The device according to claim 1, **characterised in that** the probe (10) and the signal means (7) are connected to each other by means of at least one signal wire (12).

3. The device according to any one of the preceding claims, **characterised in that** the probe (10) and the signal means (7) are connected to each other such that they cannot move in or counter to the insertion direction (V).

4. The device according to any one of the preceding claims, **characterised in that** the casing (1) guides the signal means (7) in a guiding contact along a guiding path (5, 8) extending in the insertion direction (V).

5. The device according to any one of the preceding claims, **characterised in that** the probe (10) comprises a free proximal end and a free distal end and a surface which is continuously free from the proximal end to at least close to the distal end in the insertion direction (V).

6. The device according to any one of the preceding claims, **characterised in that** the probe (10) is connected to the signal means (7) via a connecting stay which projects laterally from the probe (10).

7. The device according to any one of the preceding claims, further comprising a carrier part (6) which holds the signal means (7) and comprises a breach in the insertion direction (V) out of which the probe (10) protrudes in the insertion direction (V).

8. The device according to the preceding claim, **characterised in that** the probe (10) is fastened to the carrier part (6).

9. The device according to any one of the preceding two claims 7 and 8, **characterised in that** the probe (10) overlaps the carrier part (6) in the insertion direction (V) in a probe portion and is connected to the carrier part (6) in the region of the overlapping probe portion in a direction transverse to the insertion direction (V).

10. The device according to any one of the preceding three claims 7, 8 and 9, **characterised in that** the carrier part (6) is guided in a sliding contact with the casing (1) such that it can be moved in the insertion direction (V).

11. The device according to the preceding claim, **characterised in that** the carrier part (6) and the casing (1) form guiding paths (5, 8), situated in the sliding contact, on circumferential areas which face each other transverse to the insertion direction (V).

12. The device according to any one of the preceding claims, further comprising an insertion aid (15) for the probe (10) which overlaps the probe (10) in the insertion direction (V) over at least a substantial part of its length and can be withdrawn from the probe (10), counter to the insertion direction (V).

13. The device according to the preceding claim, **characterised in that** the insertion aid (15) comprises a cladding structure (16) which surrounds the probe (10) over at least a major part of a circumference of the probe (10).

14. The device according to the preceding claim, **characterised in that** the cladding structure (16) comprises a slit extending in the insertion direction (V), in which the probe (10) is accommodated, and **in that** the slit feeds onto an outer circumferential area of the cladding structure (16) over the entire length of the probe (10).

15. The device according to any one of the preceding three claims 12, 13 and 14, **characterised in that** the insertion aid (15) comprises an injection needle (16) which protrudes beyond the probe (10) in the insertion direction (V), and **in that** the probe (10) is arranged on or around or preferably in the injection needle (16).

16. The device according to the preceding claim, **characterised in that** the insertion aid (15) comprises at least two pivoting blades (18) which can be pivoted towards each other into a protective position, such that the injection needle (16) comes to rest between the pivoting blades (18), and **in that** the pivoting blades (18) protrude beyond a tip of the injection needle (16) in the protective position.

17. The device according to any one of the preceding claims, **characterised in that** the probe (10) is a measuring probe for recording a parameter, preferably a biochemical parameter, which characterises the state of health of an organism (glucose concentration).

18. The device according to any one of the preceding claims, **characterised by** its use for ascertaining, preferably continuously ascertaining, a parameter, preferably a biochemical parameter, which characterises the state of health of an organism (glucose concentration).

## Revendications

1. Dispositif de mise en place d'une sonde dans un tissu vivant, le dispositif comprenant,
a) un boîtier (1) avec une face inférieure (2) pour la mise en place sur le tissu,
b) la sonde (10) pouvant être déplacée par rapport au boîtier (1) au-delà de la face inférieure (2) pour une introduction dans le tissu dans une direction d'introduction (V),
c) et un dispositif de signalisation (7) pour la sonde (10), **caractérisé en ce que**
d) le dispositif de signalisation (7) est supporté de façon mobile par le boîtier (1) dans la direction d'introduction (V) jusqu'à une position finale et entraîne la sonde (10) lors d'un mouvement dans la direction d'introduction (V).

2. Dispositif selon la revendication 1, **caractérisé en ce que** la sonde (10) et le dispositif de signalisation (7) sont reliées au moyen d'au moins un câble de signalisation (12).

3. Dispositif selon l'une des revendications précédentes, **caractérisé en ce que** la sonde (10) et le dispositif de signalisation (7) sont reliées ensemble de façon immobile dans la direction d'introduction (V) et dans la direction opposée.

4. Dispositif selon l'une des revendications précédentes, **caractérisé en ce que** le boîtier (1) guide le dispositif de signalisation (7) dans un contact de guidage le long d'un rail de guidage (5, 8) s'étendant dans la direction d'introduction (V).

5. Dispositif selon l'une des revendications précédentes, **caractérisé en ce que** la sonde (10) comprend une extrémité proximale libre et une extrémité distale libre et une surface supérieure libre s'étendant de l'extrémité proximale au moins jusqu'à une proximité de l'extrémité distale dans la direction d'introduction (V).

6. Dispositif selon l'une des revendications précédentes, **caractérisé en ce que** la sonde (10) est reliée au dispositif de signalisation (7) via une barrette de liaison faisant saillie latéralement à partir de la sonde (10).

7. Dispositif selon l'une des revendications précédentes, comprenant en outre un élément de support (6) maintenant le dispositif de signalisation (7), qui comprend une brèche dans la direction d'introduction (V), à partir de laquelle la sonde (10) fait saillie dans la direction d'introduction (V).

8. Dispositif selon la revendication précédente, **caractérisé en que** la sonde (10) est fixée sur l'élément de support (6).

9. Dispositif selon l'une des deux revendications précédentes 7, 8, **caractérisé en ce que** la sonde (10) chevauche l'élément de support (6) dans la direction d'introduction (V) dans une section de sonde et est reliée dans le secteur de la section de sonde chevauchante à l'élément de support (6) dans une direction transversale par rapport à la direction d'introduction (V).

10. Dispositif selon l'une des trois revendications précédentes 7, 8, 9, **caractérisé en ce que** l'élément de support (6) est guidé de façon mobile dans un contact coulissant avec le boîtier (1) dans la direction d'introduction (V).

11. Dispositif selon la revendication précédente, **caractérisé en ce que** l'élément de support (6) et le boîtier (1) forment des faces périphériques tournées l'une vers l'autre de façon transversale par rapport à la direction d'introduction (V) dans les rails de guidage (5, 8) se trouvant en contact coulissant.

12. Dispositif selon l'une des revendications précédentes comprenant en outre une aide d'introduction (15) pour la sonde (10) qui chevauche la sonde (10) dans la direction d'introduction (V) au moins via un élément essentiel de sa longueur et qui peut être retirée dans le sens opposé à la direction d'introduction (V) de la sonde (10).

13. Dispositif selon la revendication précédente, **caractérisé en ce que** l'aide d'introduction (15) comprend une structure enveloppante (16), qui entoure la sonde (10) au moins sur une majeure partie d'une périphérie de la sonde (10).

14. Dispositif selon la revendication précédente, **caractérisé en ce que** la structure enveloppante (16) comprend une rainure s'étendant dans la direction d'introduction (V), dans laquelle la sonde (10) est reçue, et **en ce que** la rainure débouche sur la longueur totale de la sonde (10) à une surface périphérique extérieure de la structure enveloppante (16).

15. Dispositif selon l'une des trois revendications précédentes 12, 13, 14, **caractérisé en ce que** l'aide d'introduction (15) comprend une aiguille à piquer (16), qui dépasse la sonde (10) dans la direction d'introduction (V), et **en ce que** la sonde (10) est disposée sur ou autour de celle-ci ou de préférence dans l'aiguille à piquer (16).

16. Dispositif selon la revendication précédente, **caractérisé en ce que** l'aide à piquer (15) comprend au moins deux battants pivotants (18) qui peuvent être pivotés l'un sur l'autre dans une position de protection, de sorte que l'aiguille à piquer (16) vient se positionner entre les battants pivotants (18), et **en ce que** les battants pivotants (18) font saillie dans la position de protection au dessus d'une pointe de l'aiguille à piquer (16).

17. Dispositif selon l'une des revendications précédentes, **caractérisé en ce que** la sonde (10) est une sonde de mesure servant à détecter un paramètre caractérisant l'état de santé d'un organisme, de préférence un paramètre biochimique (concentration de glucose).

18. Dispositif selon l'une des revendications précédentes, **caractérisé par** l'utilisation pour la détermination, de préférence la détermination continue, d'un paramètre caractérisant l'état de santé d'un organisme, de préférence un paramètre biochimique (concentration de glucose).
